Europäisches Patentamt

European Patent Office

Office européen des brevets

(19)

(11) Numéro de publication: **0 191 688**
**B1**

(12) **FASCICULE DE BREVET EUROPEEN**

(45) Date de publication du fascicule du brevet: **08.08.90**

(51) Int. Cl.⁵: **A 61 F 9/00**

(21) Numéro de dépôt: **86400192.0**

(22) Date de dépôt: **30.01.86**

(54) **Appareil chirurgical destiné à modifier la courbure de la cornée oculaire.**

(30) Priorité: **04.02.85 FR 8501614**

(43) Date de publication de la demande:
**20.08.86 Bulletin 86/34**

(45) Mention de la délivrance du brevet:
**08.08.90 Bulletin 90/32**

(84) Etats contractants désignés:
**AT BE CH DE FR GB IT LI LU NL SE**

(56) Documents cités:
| | |
|---|---|
| EP-A-0 111 060 | US-A-3 096 767 |
| EP-A-0 151 869 | US-A-3 769 963 |
| DE-A-3 148 748 | US-A-4 461 294 |
| SU-A- 929 097 | US-A-4 538 608 |
| SU-A- 975 011 | |

AMERICAN JOURNAL OF OPHTHALMOLOGY,
vol. 96, no. 6, décembre 1983, pages 710-715;
S.L. TROKEL et al.: "Excimer laser surgery of the
cornea"

(73) Titulaire: **SYNTHELABO**
**58 rue de la Glacière**
**F-75013 Paris (FR)**

(73) Titulaire: **Azema, Alain**
**28 avenue des Arènes**
**F-06000 Nice (FR)**

(73) Titulaire: **Arneodo, Jacques**
**7 avenue Brown Séquart**
**F-06000 Nice (FR)**

(73) Titulaire: **Botineau, Jean**
**5 avenue Chateaubriand**
**F-06100 Nice (FR)**

(73) Titulaire: **Crozafon, Philippe**
**55, Promenade des Anglais**
**F-06000 Nice (FR)**

(73) Titulaire: **Moulin, Gérard**
**167, avenue Maréchal Lyautey**
**F-06000 Nice (FR)**

(72) Inventeur: **Azema, Alain**
**28 Avenue des Arènes**
**F-06000 Nice (FR)**
Inventeur: **Arneodo, Jacques**
**7 Avenue Brown Séquart**
**F-06000 Nice (FR)**
Inventeur: **Botineau, Jean**
**5 Avenue Chateaubriand**
**F-06100 Nice (FR)**

Courier Press, Leamington Spa, England.

## EP 0 191 688 B1

(56) References cited:

**HEALTH PHYSICS, vol. 40, mai 1981, pages 677-683, Health Physics Society, Pergamon Press, US; J. TABOADA et al.: "Response of the corneal epithelium to KrF excimer laser pulses"**

Inventeur: **Crozafon, Philippe**
**55 Promenade des Anglais**
**F-06000 Nice (FR)**
Inventeur: **Moulin, Gérard**
**167 Avenue Maréchal Lyautey**
**F-06000 Nice (FR)**

(74) Mandataire: **Bonnetat, Christian**
**CABINET BONNETAT 23, Rue de Léningrad**
**F-75008 Paris (FR)**

# Description

La présente invention concerne un appareil chirurgical destiné à modifier la courbure de la cornée oculaire, que cette cornée soit portée par un être vivant, ou bien ait été préalablement prélevée sur un être vivant.

On sait que certaines affections, telles que la myopie, l'hypermétropie et l'astigmatisme, peuvent être traitées par modification de la courbure de la cornée. Par ailleurs, d'autres affections, telles que l'aphaquie, peuvent être traitées au moins en partie par correction de ladite courbure. Par suite, on a déjà pensé à des méthodes permettant de modifier la courbure de la cornée.

Ces méthodes connues sont essentiellement de deux types:

le premier consiste à prélever, à l'aide d'un instrument mécanique coupant, une lentille plan-convexe de la cornée, à congeler cette lentille pour la rendre solide et à usiner, au moyen d'un tour, ladite lentille solidifiée afin d'en éliminer une zone en forme de lamelle bombée d'épaisseur variable. Après usinage, la lentille usinée est décongelée, puis recousue à son emplacement initial. Une telle méthode est longue, onéreuse et traumatisante pour le patient. De plus, elle ne permet de soigner que des affections de révolution autour de l'axe optique de l'oeil, telles que la myopie et l'hypermétropie, et non pas l'astigmatisme qui ne présente pas de symétrie de révolution;

le second type de méthodes a pour mode opératoire de pratiquer dans la cornée, à l'extérieur de la zone optique, des fentes radiales au moyen d'un bistouri ou d'un faisceau laser. A cause de ces fentes, la partie de cornée incisée s'aplatit, modifiant de ce fait la courbure générale. Quoique moins traumatisantes que les méthodes du premier type, celles du second type restent violentes à cause des scarifications obligatoires de la cornée. De plus, les possibilités de ces dernières méthodes restent limitées.

Par ailleurs, dans l'article "Excimer laser surgery of the cornea" daté du 21 Septembre 1983 et paru dans la revue "AMERICAN JOURNAL OF OPHTHALMOLOGY" Vol. 96 n° 6, pages 710 à 715 de Décembre 1983, Stephen L. TROKEL, R. SRINI-VASAN et BODIL BRAREN décrivent l'utilisation d'un laser à excimère pour éliminer la matière cornéenne par photodécomposition. Dans cet article, il est recommandé d'utiliser un rayonnement ultraviolet dont la longueur d'onde est égale à 0,193 micromètre et il est précisé que, pour modifier la courbure de la cornée, on peut former sur ladite cornée, à l'aide dudit rayonnement, une tache circulaire dont "l'intensité varie du centre vers la périphérie, de sorte que l'on enlève plus de matière soit au centre, soit à la périphérie, en fonction de la distribution de lumière". On peut ainsi augmenter ou diminuer la courbure de la cornée.

Cette dernière méthode est particulièrement intéressante, mais elle est difficile à mettre en oeuvre, car elle nécessite des moyens permettant de faire varier l'intensité lumineuse en correspondance avec la courbure cornéenne désirée. La réalisation technologique de tels moyens paraît difficile, pour ne pas dire impossible.

On remarquera que les documents US—A—4 538 608 et EP—A—0 151 869 décrivent des dispositifs mettant en oeuvre un faisceau laser ablatif ponctuel, animé d'un mouvement de balayage. De tels dispositifs présentent l'inconvénient que la surface de cornée corrigée n'est pas uniforme, car obtenue par une succession d'enlèvements ponctuels.

La présente invention a pour objet de remédier aux inconvénients de ces méthodes connues. Elle concerne un appareil chirurgical permettant de modifier la courbure de la cornée de façon non traumatisante, rapide et aisée pour traiter des affections aussi différentes que la myopie, l'hypermétropie, l'aphaquie, et l'astigmatisme.

A ces fins, selon l'invention, l'appareil chirurgical destiné à modifier au moins en partie la courbure de la cornée oculaire par ablation d'une zone de celle-ci en forme de lamelle lenticulaire d'épaisseur radialement variable, et comportant une source de lumière susceptible d'émettre un faisceau dont la longueur d'onde est voisine de 0,2 micromètre pour permettre la photodécomposition de la matière cornéenne, ainsi qu'un système optique dirigeant ledit faisceau sur ladite zone de cornée à éliminer et y formant une tache lumineuse sur une partie de ladite zone, est remarquable en ce que ledit système optique forme sur la cornée une tache constamment centrée sur l'axe optique de l'oeil et en ce qu'il comporte des moyens d'exploration pour faire varier progressivement l'aire de cette tache sur la cornée, de façon que les parties de ladite zone à éliminer soient d'autant plus longtemps exposées audit faisceau qu'elles correspondent à des épaisseurs plus grandes de ladite lamelle lenticulaire et vice-versa.

Ainsi, on peut obtenir l'ablation de la matière cornéenne sans scarification par un processus photochimique, particulièrement peu traumatisant pour le patient sans avoir à concevoir des moyens susceptibles de faire varier radialement l'intensité du faisceau laser.

En effet, puisque selon l'invention, on fait en sorte que les parties de la cornée correspondant à de grandes épaisseurs à éliminer soient plus exposées au faisceau lumineux que les parties de cette même cornée correspondant à de petites épaisseurs à éliminer, on obtient une ablation photochimique différentielle de la cornée, permettant de modifier la courbure de celle-ci, puisque ces parties épaisses reçoivent plus d'énergie lumineuse que ces parties minces.

La progressivité de cette ablation photochimique différentielle dépend alors uniquement de la progressivité de l'exploration de la totalité de la zone à éliminer par ladite tache lumineuse.

Pour obtenir le maximum de progressivité d'exploration et donc de précision dans la modification de la courbure de la cornée, on prévoit essentiellement, selon l'invention, que la tache lumineuse étant constamment centrée sur l'axe optique de l'oeil l'appareil conforme à l'invention comporte des moyens d'exploration pour faire varier progressivement l'aire de cette tache sur la cornée. Ainsi, l'invention s'applique pratiquement à tous les traitements. En effet:

a) le traitement de la myopie — corrigible par une réduction de la courbure de la cornée — nécessite l'ablation d'une lamelle en forme de lentille, plus épaisse en son centre (voisinage de l'axe optique de l'oeil) qu'à sa périphérie. Par suite, dans ce cas, il faut que la densité d'énergie lumineuse reçue par la cornée aille en décroissant du centre vers la périphérie, ou en croissant de la périphérie vers le centre.

Pour ce faire, le système optique de l'appareil conforme à l'invention forme, sur la cornée, une tache lumineuse circulaire et lesdits moyens d'exploration font varier progressivement l'aire de ladite tache depuis une petite tache centrale jusqu'à l'aire totale de la zone à éliminer, ou vice-versa. Dans le cas où la variation d'aire de ladite tache s'effectue dans le sens de l'agrandissement, c'est-à-dire du centre vers la périphérie, la durée d'exposition au faisceau lumineux décroît au fur et à mesure que l'on s'éloigne du centre. Au contraire, lorsque l'exploration de la zone à éliminer a lieu depuis la périphérie vers le centre, la durée d'exposition au faisceau lumineux croît au fur et à mesure que l'on se rapproche du centre. Bien entendu, dans l'un et l'autre cas, la variation de la durée d'exposition est réglée pour respecter la variation de courbure désirée.

b) Le traitement de l'hypermétropie et de l'aphaquie — corrigibles par une augmentation de la courbure de la cornée — nécessite l'ablation d'une lamelle plus épaisse à sa périphérie qu'au centre (voisinage de l'axe optique). Par suite, dans ce cas, il faut que la densité d'énergie lumineuse reçue par la cornée aille en croissant du centre vers la périphérie ou en décroissant de la périphérie vers le centre.

Pour ce faire, le système optique de l'appareil conforme à l'invention forme, sur la cornée, une tache lumineuse annulaire et lesdits moyens d'exploration font varier progressivement l'aire de ladite tache, au moins par variation du diamètre intérieur de celle-ci. Bien entendu, cette variation d'aire de la tache annulaire est réglée pour que l'on obtienne la modification de profil désirée. Elle peut s'effectuer aussi bien dans le sens de l'agrandissement que dans celui de la réduction.

c) Le traitement de l'astigmatisme (dû à des rayons de courbure différents de la cornée dans deux plans différents) nécessite une correction de courbure suivant un plan méridien particulier passant par l'axe optique. Aussi, dans ce cas, le système optique de l'appareil conforme à l'invention forme sur la cornée une tache lumineuse allongée dont les grands côtés rectilignes sont perpendiculaires audit méridien particulier et lesdits moyens d'exploration font varier progressivement l'aire de ladite tache, au moins par variation de la longueur des petits côtés de celle-ci.

Là encore, la variation d'aire peut résulter d'un agrandissement ou d'une réduction, du moment que ladite variation soit telle (en fonction du temps) qu'elle communique à la cornée la modification de courbure désirée.

Dans une forme préférentielle de réalisation de l'appareil chirurgical conforme à l'invention, lesdits moyens d'exploration comportent au moins un écran ou un diaphragme et des moyens de déplacement pour engendrer un déplacement relatif entre ledit système optique et au moins l'un desdits écrans ou diaphragmes, la vitesse de ce déplacement relatif correspondant à la variation radiale d'épaisseur de ladite lamelle à éliminer. De préférence, la direction de ce déplacement relatif est parallèle audit faisceau.

Avantageusement, ledit système optique et lesdits écrans et/ou diaphragmes sont montés sur un banc optique.

Ainsi, pour traiter la myopie, l'appareil selon l'invention comporte:

soit un diaphragme fixe et un système optique mobile, recevant le faisceau issu dudit diaphragme;

soit un premier diaphragme fixe, un système optique également fixe recevant le faisceau issu dudit premier diaphragme et un second diaphragme mobile, disposé du côté opposé au premier par rapport audit système optique.

De façon analogue, pour traiter l'hypermétropie et l'aphaquie, l'appareil selon l'invention comporte:

soit un diaphragme fixe, un système optique mobile recevant le faisceau issu dudit diaphragme et un écran, qui est solidaire en déplacement dudit système optique et qui occulte la partie centrale du faisceau traversant celui-ci;

soit un diaphragme fixe, un système optique également fixe recevant le faisceau issu dudit diaphragme et un écran mobile, qui occulte la partie centrale du faisceau traversant ledit système optique.

Pour traiter l'astigmatisme, l'appareil selon l'invention peut comporter un diaphragme fixe, un système optique également fixe et un écran mobile percé d'une fente. Cette fente est transversale à la direction de déplacement dudit écran qui peut être déplacé parallèlement à lui-même. L'appareil peut comporter des moyens pour régler l'orientation de ladite fente dans le plan dudit écran mobile.

Afin d'obtenir un appareil chirurgical automatique, il est avantageux de prévoir un calculateur électronique commandant ladite source de lumière et lesdits moyens d'exploration en fonction de la variation radiale d'épaisseur désirée pour ladite lamelle à éliminer. L'appareil comporte alors de plus un kératomètre susceptible de suivre la modification de courbure de la cornée et d'adresser ses mesures audit calculateur. On prévoit également, afin de faciliter l'alignement du faisceau d'ablation par rapport à ladite cornée, un générateur laser auxiliaire, par exemple du type hélium-néon.

Les figures du dessin annexé feront bien comprendre comment l'invention peut être réalisée. Sur ces figures, des références identiques désignent des éléments semblables ou analogues.

Les figures 1 et 2 illustrent schématiquement, respectivement en vue axiale et en vue de face, le principe selon lequel fonctionne l'appareil de l'invention, dans le cas du traitement de la myopie.

Les figures 3 et 4 montrent les schémas de deux modes de réalisation d'une partie de l'appareil conforme à l'invention, lorsqu'il est destiné au traitement de la myopie.

Les figures 5 et 6 illustrent schématiquement, respectivement en vue axiale et en vue de face, le principe selon lequel fonctionne l'appareil de l'invention, dans le cas du traitement de l'hypermétropie et de l'aphaquie.

Les figures 7 et 8 montrent des schémas de deux modes de réalisation d'une partie de l'appareil conforme à l'invention, lorsqu'il est destiné au traitement de l'hypermétropie et de l'aphaquie.

La figure 9 montre schématiquement un mode de réalisaton d'une partie de l'appareil conforme à l'invention, lorsqu'il est destiné au traitement de l'astigmatisme.

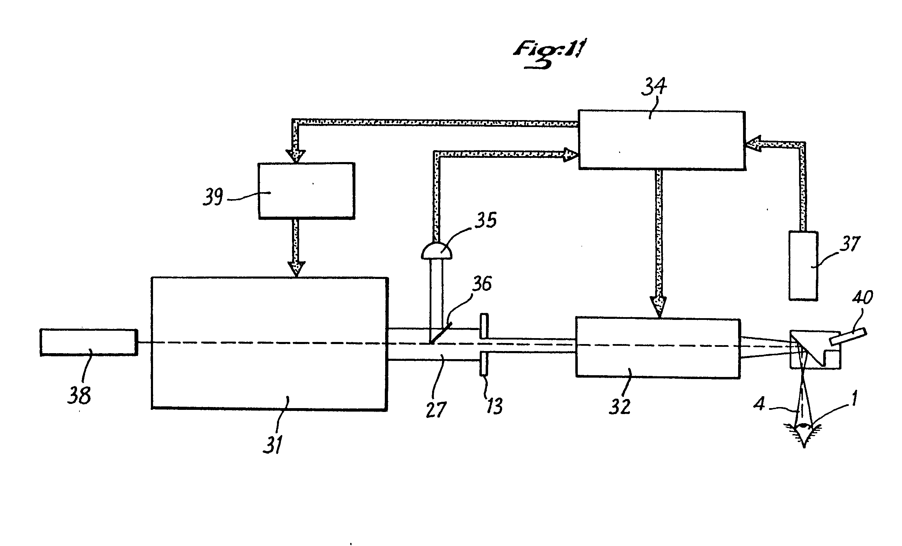

La figure 10 illustre schématiquement le fonctionnement du mode de réalisation de la figure 9.

La figure 11 est le schéma synoptique d'ensemble de l'appareil conforme à l'invention.

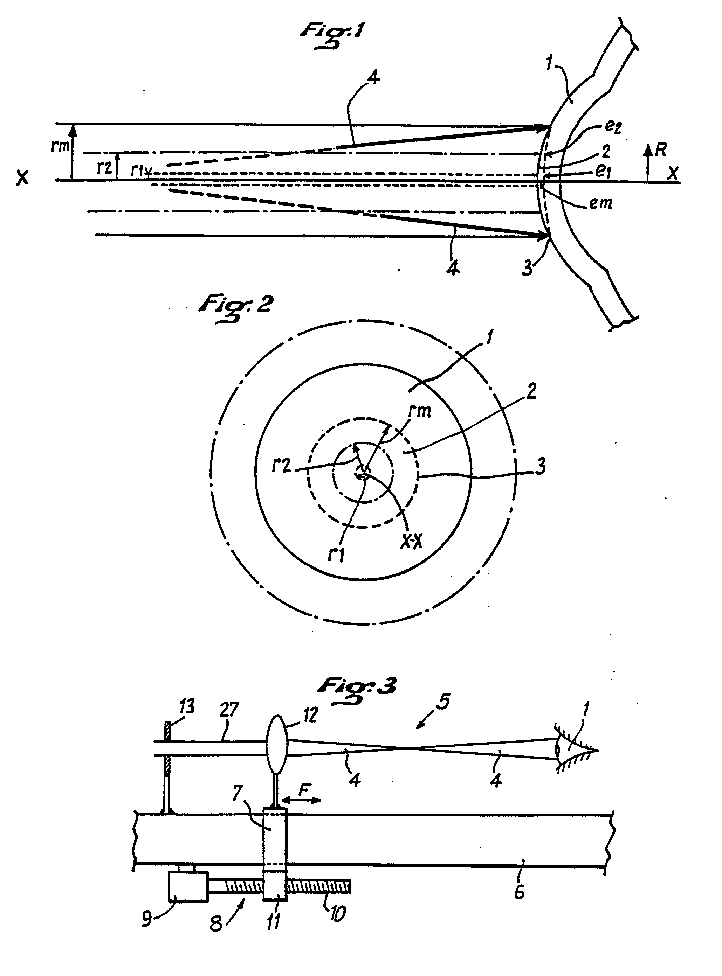

Sur les figures 1 et 2, on a illustré le principe selon lequel fonctionne l'appareil conforme à la présente invention, dans le cas du traitement de la myopie.

Sur ces figures, on a représenté schématiquement, respectivement en coupe axiale et en vue de face, la cornée 1 d'un oeil myope.

De façon connue, on sait qu'un tel défaut peut être corrigé chirurgicalement en éliminant une lamelle 2 en forme de lentille circulaire, de façon à réduire ladite courbure.

Cette lamelle lenticulaire 2 est centrée sur l'axe optique X—X de l'oeil et présente en son centre une épaisseur maximale $em$, qui va en décroissant vers sa périphérie 3, en fonction de la distance radiale R à l'axe X—X.

La progressivité de la variation de l'épaisseur $e$ de ladite lamelle 2 en fonction de la variation de la distance radiale R est déterminée par la correction de courbure désirée.

Selon l'invention, pour obtenir l'ablation de la lamelle 2 présentant la loi e (R) de variation d'épaisseur souhaitée en fonction de ladite distance radiale, on utilise un faisceau lumineux 4 de section circulaire, dont la longueur d'onde est au plus égale à 0,2 micromètre et qui présente une densité d'énergie constante, transversalement à sa direction. Le faisceau lumineux 4 est dirigé sur la cornée 1 et centré sur l'axe optique X—X.

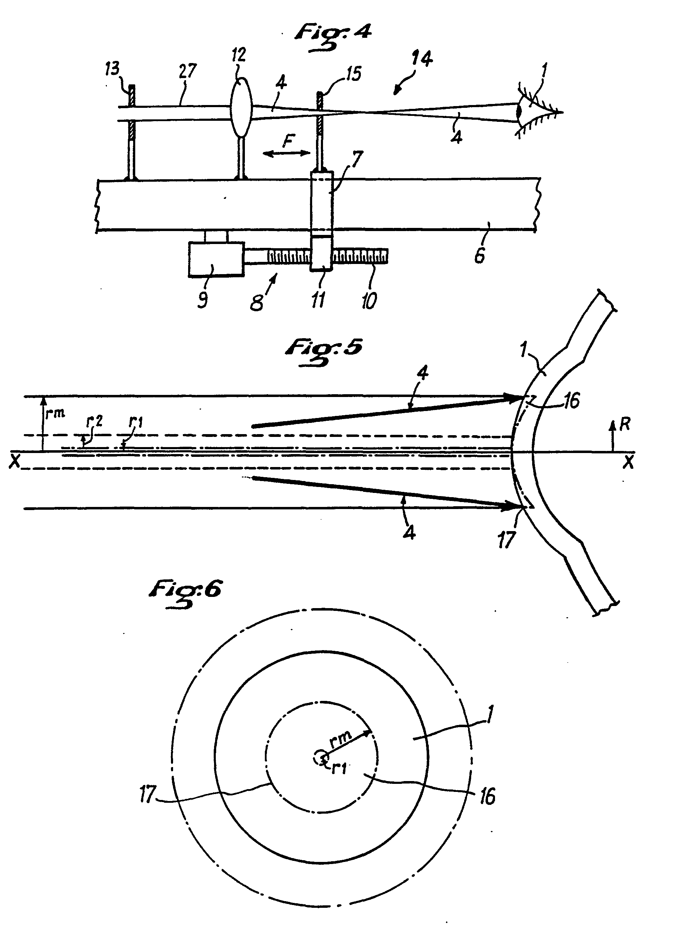

Grâce à des moyens décrits en regard des figures 3 et 4 on fait varier, en fonction du temps $t$, le rayon $r$ de la tache formée par ledit faisceau 4 sur la cornée 1 suivant une loi permettant de satisfaire la loi e (R) désirée, c'est-à-dire telle que, pour chaque valeur 0, . . . , r1, . . . , r2, . . . , rm du rayon de ladite tache, on obtient une profondeur d'élimination de cornée correspondant aux épaisseurs em, . . . , e1, . . . e2, . . . 0 de ladite lamelle 2.

Bien entendu, la variation r (t) du rayon $r$ en fonction du temps $t$ peut être réalisée depuis la valeur maximale rm du rayon r vers zéro, ou bien depuis zéro vers la valeur maximale rm.

On voit ainsi que, selon l'invention, l'ablation de la lamelle 2 désirée est obtenue en formant, sur la cornée 1, une tache lumineuse circulaire et en faisant varier le rayon de cette tache en fonction du temps, afin d'obtenir la loi e (R) désirée.

Pour obtenir cette tache lumineuse de diamètre variable, appropriée au traitement de la myopie, on peut mettre en oeuvre l'un ou l'autre des dispositifs montrés schématiquement sur les figures 3 et 4.

Les dispositifs 5, montré par la figure 3, comporte un banc optique 6 le long duquel peut se déplacer dans les deux sens (flèches F) un chariot 7, sous l'action de moyens d'entraînement 8, comprenant par exemple un moteur électrique 9 entrainant en rotation une vis sans fin 10, coopérant avec un écrou 11 lié audit chariot 7.

Le chariot 7 porte un système optique représenté sous forme d'une lentille 12 engendrant le faisceau lumineux 4 et susceptible de former une tache lumineuse sur la cornée 1. En faisant varier la position de la lentille 12, grâce au chariot 7, on fait varier le rayon de ladite tache sur ladite cornée et le contrôle de la vitesse de déplacement du chariot 7 le long du banc 6, par action des moyens 8, permet le contrôle de la loi de variation r (t) et donc de la loi e (R).

Un diaphragme fixe 13 permet d'adresser à la lentille 12 un faisceau lumineux 27 parallèle homogène et de section circulaire, duquel est issue le faisceau 4.

Dans la variante de réalisation 14 montrée par la figure 4, la lentille 12 est montée à poste fixe sur le banc 6. En revanche, on prévoit un diaphragme circulaire supplémentaire 15 monté sur le chariot 7. En actionnant les moyens 8, on déplace le diaphragme 15 le long du banc 6 et, en conséquence, on fait varier les dimensions de la tache formée par le faisceau 4 sur la cornée 1.

En se reportant maintenant aux figures 5 et 6, on explique le principe selon lequel fonctionne l'appareil conforme à la présente invention pour le traitement de l'hypermétropie et l'aphaquie. Dans ce cas, on sait que de tels défauts peuvent être corrigées chirurgicalement en éliminant une lamelle 16 en forme de ménisque moins épais au centre qu'au bord, de façon à augmenter localement la courbure de la cornée.

La lamelle 16 est centrée sur l'axe optique X—X de l'oeil et présente un épaisseur qui va en croissant vers sa périphérie 17.

Par analogie avec ce qui a été décrit en regard des figures 1 et 2, on comprendra aisément que, à l'aide du faisceau lumineux 4 formant alors une tache annulaire sur la cornée 1, on puisse faire l'ablation de la lamelle 16, en faisant varier le rayon intérieur de ladite tache en fonction du temps, afin de respecter la loi de variation de l'épaisseur de ladite lamelle 16, parallèlement à la direction radiale R.

Dans ce cas, à l'inverse de ce qui a été mentionné pour le traitement de la myopie, la durée d'exposition au faisceau 4 doit durer d'autant plus longtemps que l'on s'éloigne de l'axe optique X—X.

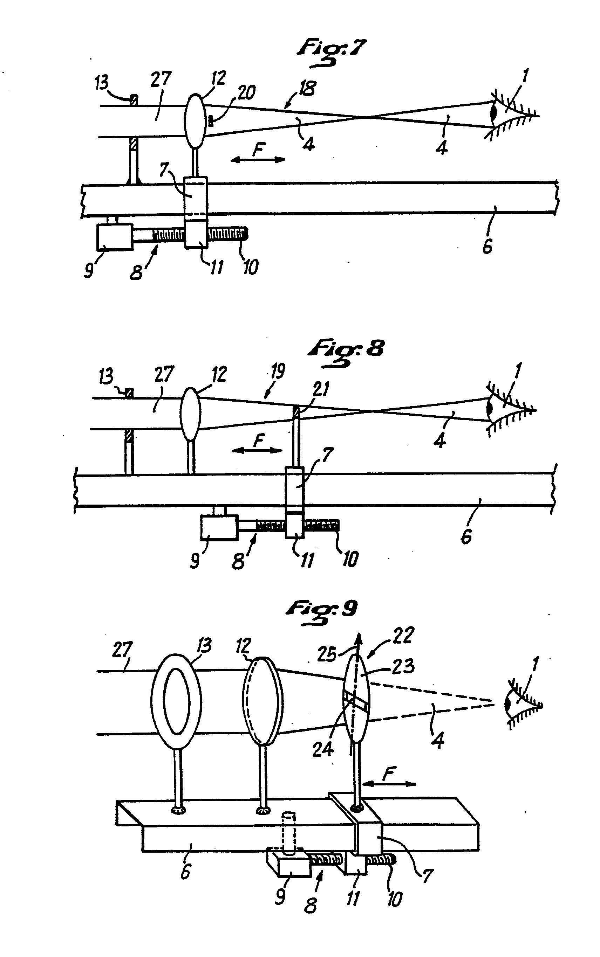

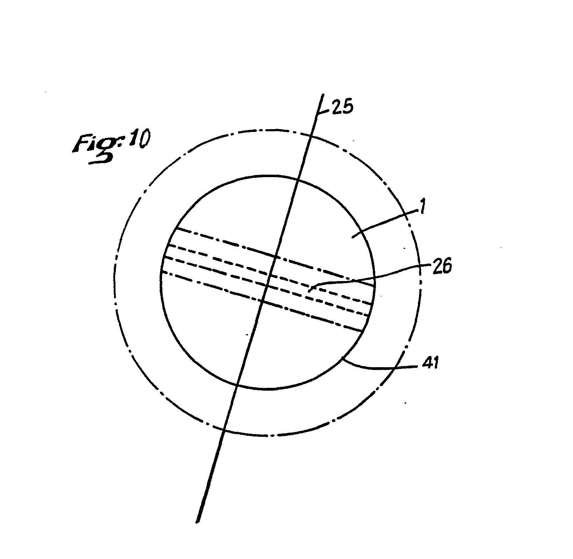

Pour mettre en oeuvre le processus illustré par les figures 5 et 6, on peut utiliser l'un ou l'autre des dispositifs 18 et 19 des figures 7 ou 8.

Le dispositif 18 est identique au dispositif 5 de la figure 3, à la différence près que la partie centrale de la lentille 12 est occultée par un écran central circulaire 20. Par suite, lorsque les moyens d'entraînement 8 déplacent la lentille 12 le long du banc 6, il en résulte une variation de dimension de l'ombre portée par ledit écran 20 sur la partie centrale de ladite cornée 1. En ajustant la vitesse de déplacement de l'ensemble 12, 20 le long du banc 6 (grâce à la commande des moyens 8), on règle donc le temps d'exposition de la cornée 1 au faisceau 4, en fonction de la distance radiale, afin d'obtenir l'ablation de la lamelle 16 de profil désiré.

Un résultat identique est obtenu par la mise en oeuvre du dispositif 19, qui est semblable au dispositif 14 de la figure 4, sauf en ce qui concerne le diaphragme 15 monté sur le chariot 7. Ce diaphragme 15 est remplacé par un écran circulaire 21 occultant la partie centrale du faisceau 4. Le déplacement du chariot 7 entraîne alors la variation d'aire de l'ombre, portée par ledit écran 21 sur la partie centrale de la cornée 1.

Sur la figure 9, on a représenté partiellement et schématiquement en perspective un dispositif 22 conforme à l'invention, destiné à corriger l'astigmatisme. Ce dispositif 22 est identique au dispositif 14 de la figure 4, à l'exception du fait que la diaphragme circulaire 15 est remplacé par un écran 23, percé d'une fente rectiligne 24. La fente rectiligne 24 est orthogonale à une direction prédéterminée 25.

Ainsi, comme l'illustre la figure 11, on forme sur la cornée une tache lumineuse allongée 26 dont les grands côtés sont rectilignes et orthogonaux à ladite directon prédéterminée 25, qui correspond à la trace du méridien de l'oeil suivant lequel doit être effectuée la correction de courbure. Latéralement, la tâche 26 est limitée par la portion correspondante de la ligne circulaire 41 déterminée par le diaphragme 13.

Lors du déplacement du chariot 7 et de l'écran 23 (sous l'action des moyens 8 à 11 non représentés sur la figure 9) le long du banc 6, l'aire de la tache 26 sur la cornée 1 croît et/ou décroît en fonction du sens dudit déplacement, de sorte qu'il est possible de faire l'ablation d'une lamelle correspondant à la correction désirée.

On voit ainsi que les dispositifs 5, 14, 18, 19 et 22 représentés et décrits en regard des figures 3, 4, 7, 8 et 9 explorent la cornée 1 au moyen d'une variation de l'aire d'une tache lumineuse.

Sur la figure 11, on a représenté le schéma synoptique d'un appareil automatique conforme à la présente invention.

Cet appareil comporte:

une source lumineuse 31 susceptible d'émettre un faisceau lumineux homogène 27, dont la longueur d'onde est au plus égale à 0,2 micromètre, par exemple un générateur laser à excimère, du type à mélange argon-fluor;

un système 32 dirigeant ledit faisceau lumineux, après passage à travers le diaphragme 13, vers la cornée 1 et comportant des moyens d'exploration de celle-ci; le système 32 peut être l'un ou l'autre des dispositifs 5, 14, 18, 19, 22 décrits ci-dessus, ou bien encore un dispositif analogue;

un calculateur électronique 34 chargé de piloter le processus de fonctionnement dudit appareil et notamment de commander le dispositif 32 (plus spécialement les moyens d'actionnement 8);

un photodétecteur 35, associé à un miroir semi-transparent 36, et destiné à fournir au calculateur 34 des informations relatives à l'énergie des impulsions du faisceau 4;

un kératomètre automatique 37 qui mesure en temps réel la courbure de la cornée 1 et transmet ses mesures au calculateur 34;

un laser d'alignement 38, par exemple du type hélium-néon, permettant un positionnement correct du faisceau 4 sur la cornée 1;

un dispositif 39 de commande de la source 31, lui-même commandé par le calculateur 34; et,

une lampe à fente 40 permettant au chirurgien l'observation de la cornée 1 en cours d'opération.

Le calculateur 34 est programmé pour commander le dispositif 32 en fonction de la loi de variation d'épaisseur désirée pour la lamelle à éliminer de la cornée 1 et il exécute les séquences d'operations correspondantes en prenant en compte les informations qui lui sont fournies par le kératomètre 37 et le photodétecteur 35. Le dispositif 39 comporte des moyens d'arrêt automatique d'urgence, en cas de nécessité.

## Revendications

1. Appareil chirurgical destiné à modifier au moins en partie la courbure de la cornée oculaire (1) par ablation d'une zone de celle-ci en forme de lamelle lenticulaire (2, 16) d'épaisseur radialement variable, et comportant une source de lumière (31) susceptible d'émettre un faisceau dont la longueur d'onde est voisine de 0,2 micromètre pour permettre la photodécomposition de la matière cornéenne, ainsi qu'un système optique (12) dirigeant ledit faisceau sur ladite zone de cornée à éliminer et y formant une tache lumineuse sur une partie de ladite zone, caractérisé en ce que ledit système optique (12) forme sur la cornée une tache constamment centrée sur l'axe optique de l'oeil et en ce qu'il comporte des moyens d'exploration (8, 13, 15, 20, 21, 23) pour faire varier progressivement l'aire de cette tache sur la cornée, de façon que les parties de ladite zone a éliminer soient d'autant plus longtemps exposées audit faisceau qu'elles correspondent à des épaisseurs plus grandes de ladite lamelle lenticulaire et vice-versa.

2. Appareil selon la revendication 1, caractérisé en ce que ladite tache lumineuse est circulaire.

3. Appareil selon la revendication 1, caractérisé en ce que ladite tache lumineuse est annulaire.

4. Appareil selon la revendication 1, caractérisé en ce que ladite tache lumineuse est sensiblement rectangulaire.

5. Appareil selon l'une quelconque des revendications 1 à 4, caractérisé en ce que lesdits moyens d'exploration comportent au moins un écran ou diaphragme (13, 15, 20, 21, 23) et des moyens de déplacement (8, 30) pour engendrer un déplacement relatif entre ledit système optique (12) et au moins l'un desdits écrans ou diaphragmes, la vitesse de ce déplacement relatif correspondant à la variation d'épaisseur de ladite lamelle à éliminer (2, 16).

6. Appareil selon la revendication 5, caractérisé en ce que lesdits moyens de déplacement (8) engendrent un déplacement relatif de direction parallèle audit faisceau (4).

7. Appareil selon l'une quelconque des revendications 5 ou 6, caractérisé en ce que ledit système optique (12) et lesdits écrans (13, 15, 20, 21, 23) sont montés sur un banc optique (6).

8. Appareil selon la revendication 6, caractérisé en ce qu'il comporte un diaphragme (13) fixe et un système optique (12) mobile, recevant un faisceau parallèle issu dudit diaphragme.

9. Appareil selon la revendication 6, caractérisé en ce qu'il comporte un premier diaphragme (13) fixe, un système optique (12) également fixe recevant le faisceau parallèle issu dudit premier diaphragme et un second diaphragme (15) mobile, disposé du côté opposé au premier par rapport audit système optique.

10. Appareil selon la revendication 6, caractérisé en ce qu'il comporte un diaphragme (13) fixe, un système optique (12) mobile recevant le faisceau parallèle issu dudit diaphragme et un écran (20), qui est solidaire en déplacement dudit système optique et qui occulte la partie centrale du faisceau (4) traversant celui-ci.

11. Appareil selon la revendication 6, caractérisé en ce qu'il comporte un diaphragme (13) fixe, un système optique (12) également fixe recevant le faisceau parallèle issu dudit diaphragme et un écran (21) mobile, qui occulte la partie centrale du faisceau (4) traversant ledit système optique.

12. Appareil selon la revendication 6, caractérisé en ce qu'il comporte un diaphragme (13) fixe, un système optique (12) également fixe et un écran (23) mobile et percé d'une fente (24).

13. Appareil selon l'une quelconque des revendications 1 à 12, caractérisé en ce qu'il comporte un calculateur électronique (34) commandant ladite source de lumière (31) et lesdits moyens d'exploration (8, 13, 15, 20, 21, 23) en fonction de la variation d'épaisseur désirée pour ladite lamelle à éliminer (2, 16).

14. Appareil selon la revendication 13, caractérisé en ce qu'il comporte de plus un kératomètre (37) susceptible de suivre en continu l'évolution de la courbure de la partie de la cornée éliminée et d'adresser ses mesures audit calculateur (34).

15. Appareil selon l'une quelconque des revendications 13 ou 14, caractérisé en ce qu'il comporte un générateur laser auxiliaire (38) destiné à alignement dudit faisceau (4) par rapport à ladite cornée (1).

## Patentansprüche

1. Chirurgische Vorrichtung zur Berichtigung zumindest eines Teils der Krümmung der Hornhaut (1) durch Abtragen einer Hornhautzone als linsenförmige Lamelle (2, 16) von radial veränderlicher Dicke und bestehend aus einer Lichtquelle (31), von der ein Strahlenbündel ausgesendet werden kann, dessen Wellenlänge bei annähernd 0,2 Mikrometer liegt, um die Photozersetzung der Hornhautsubstanz zu ermöglichen, sowie aus einem optischen System (12), durch das das Strahlenbündel auf die zu entfernende Hornhautzone gerichtet wird und das dort auf einem Teil der Zone einen Leuchtfleck bildet, dadurch gekennzeichnet, daß das optische System (12) auf der Hornhaut einen beständig auf der optischen Achse des Auges zentrierten Fleck bildet und daß es Abtastmittel (8, 13, 15, 20, 21, 23) zum fortschreitenden Variieren der Fläche des Flecks auf der Hornhaut aufweist, so daß die Teile der zu entfernenden Zone um so länger dem Strahlenbündel ausgesetzt werden, wie diese der größeren Dicke der linsenförmigen Lamelle entsprechen und umgekehrt.

2. Vorrichtung nach Anspruch 1, dadurch gekennzeichnet, daß der Leuchtfleck kreisförmig ist.

3. Vorrichtung nach Anspruch 1, dadurch gekennzeichnet, daß der Leuchtfleck ringförmig ist.

4. Vorrichtung nach Anspruch 1, dadurch gekennzeichnet, daß der Leuchtfleck allgemein rechteckförmig ist.

5. Vorrichtung nach einem der vorhergehenden Ansprüche 1 bis 4, dadurch gekennzeichnet, daß die Abtastmittel zumindest eine Blende oder ein Diaphragma (13, 15, 20, 21, 23) sowie Verstellmittel (8) aufweist, um ein relatives Versetzen zwischen dem optischen System (12) und zumindest einer der Blenden oder Diaphragmen zu erzeugen, wobei die Geschwindigkeit der relativen Versetzung der Veränderung oder Schwankung in der Dicke der zu entfernenden Lamelle (2, 16) entspricht.

6. Vorrichtung nach Anspruch 5, dadurch gekennzeichnet, daß die Verstellmittel (8) eine relative Versetzung in paralleler Richtung zum Strahlenbündel (4) erzeugen.

7. Vorrichtung nach einem der Ansprüche 5 oder 6, dadurch gekennzeichnet, daß das optische System (12) und die Blenden (13, 15, 20, 21, 23) auf einer optischen Bank oder Reihe (6) montiert sind.

8. Vorrichtung nach Anspruch 6, dadurch gekennzeichnet, daß sie ein festes Diaphragma (13) und ein bewegliches optisches System (12) aufweist, das ein vom Diaphragma abgeleitetes paralleles Strahlenbündel empfängt.

9. Vorrichtung nach Anspruch 6, dadurch gekennzeichnet, daß sie ein erstes festes Diaphragma (13), ein gleichfalls festes optisches System (12), das das vom ersten Diaphragma abgeleitete parallele Strahlenbündel empfängt, und ein zweites bewegliches Diaphragma (15) aufweist, das zum ersten auf der gegenüberliegenden Seite gegenüber dem optischen System angeordnet ist.

10. Vorrichtung nach Anspruch 6, gekennzeichnet durch ein festes Diaphragma (13), ein bewegliches, das vom Diaphragma abgeleitete parallele Strahlenbündel empfangendes optisches System (12) und eine Blende (20), die bei der Versetzung des optischen Systems mit diesem verbunden ist und den das optische System durchlaufenden zentralen Teil des Strahlenbündels (4) verdeckt.

11. Vorrichtung nach Anspruch 6, gekennzeichnet durch ein festes Diaphragma (13), ein gleichfalls festes optisches System (12) das das vom Diaphragma abgeleitete parallele Strahlenbündel empfängt, und eine bewegliche Blende (21), die den das optische System durchlaufenden zentralen Teil des Strahlenbündels (4) verdeckt.

12. Vorrichtung nach Anspruch 6, gekennzeichnet durch ein festes Diaphragma (13), ein gleichfalls festes optisches System (12) und eine bewegliche, von einem Spalt durchbrochene Blende (24).

13. Vorrichtung nach einem der vorhergehenden Ansprüche 1 bis 12, gekennzeichnet durch einen elektronischen Rechner (34), von dem die Lichtquelle (31) und die Abtastmittel (8, 13, 15, 20, 21, 23) in Abhängigkeit von der Veränderung oder Schwankung in der gewünschten Dicke für die zu entfernende Lamelle (2, 16) gesteuert wird.

14. Vorrichtung nach Anspruch 13, gekennzeichnet durch ein Keratometer (37), das in der Lage ist, die Krümmungsentwicklung des Teils der entfernten Hornhaut kontinuierlich zu verfolgen und deren Meßwerterfassung an den Rechner (34) zu adressieren.

15. Vorrichtung nach einem der vorhergehenden Ansprüche 13 oder 14, gekennzeichnet durch einen mitwirkenden Lasergenerator (38), der zum Ausrichten des Strahlenbündels (4) gegenüber der Hornhaut (1) dient.

**Claims**

1. Surgical apparatus for modifying at least partly the curvature of the eye cornea (1) by ablation of a zone thereof in the form of a lenticular lamina (2, 16) of radially variable thickness, and comprising a source of light (31) capable of emitting a beam of which the wavelength is close to 0.2 micrometre to allow photodecomposition of the corneal matter, as well as an optical system (12) directing said beam on said zone of cornea to be eliminated and forming a light spot on part of said zone, characterized in that said optical system (12) forms on the cornea a spot constantly centred on the optical axis of the eye and in that it comprises scanning means (8, 13, 15, 20, 21, 23) for progressively varying the area of this spot on the cornea, so that the parts of said zone to be eliminated are exposed to said beam the longer as they correspond to larger thicknesses of said lenticular lamina, and vice versa.

2. Apparatus according to claim 1, characterized in that said light spot is circular.

3. Apparatus according to claim 1, characterized in that said light spot is annular.

4. Apparatus according to claim 1, characterized in that said light spot is substantially rectangular.

5. Apparatus according to any one of claims 1 to 4, characterized in that said scanning means comprise at least one screen or diaphragm (13, 15, 20, 21, 23) and displacement means (8) for creating a relative displacement between said optical system (12) and at least one of said screens or diaphragms, the speed of this relative displacement corresponding to the variation in thickness of said lamina (2, 16) to be eliminated.

6. Apparatus according to claim 5, characterized in that said displacement means (8) create a relative displacement of direction parallel to said beam (4).

7. Apparatus according to any one of claims 5 or 6, characterized in that said optical system (12) and said screens (13, 15, 20, 21, 23) are mounted on an optical bench (6).

8. Apparatus according to claim 6, characterized in that it comprises a fixed diaphragm (13) and a mobile optical system (12), receiving a parallel beam issuing from said diaphragm.

9. Apparatus according to claim 6, characterized in that it comprises a first fixed diaphragm (13), a likewise fixed optical system (12) receiving the parallel beam issuing from said first diaphragm and a second mobile diaphragm (15), disposed on the side opposite the first with respect to said optical system.

10. Apparatus according to claim 6, characterized in that it comprises a fixed diaphragm (13), a mobile optical system (12) receiving the parallel beam issuing from said diaphragm and a screen (21), which is fast in displacement with said optical system and which occults the central part of the beam (4) passing therethrough.

11. Apparatus according to claim 6, characterized in that it comprises a fixed diaphragm (13), a likewise fixed optical system (12) receiving the parallel beam issuing from said diaphragm and a mobile screen (21), which occults the central part of the beam (4) passing through said optical system.

12. Apparatus according to claim 6, characterized in that it comprises a fixed diaphragm (13), a likewise fixed optical system (12) and a mobile screen (23) pierced with a slot (24).

13. Apparatus according to any one of claims 1 to 12, characterized in that it comprises an electronic computer (34) controlling said source of light (31) and said scanning means (8, 13, 15, 20, 21, 23) as a function of the desired variation in thickness for said lamina (2, 16) to be eliminated.

14. Apparatus according to claim 13, characterized in that it further comprises a keratometer (37) capable of continuously following the evolution of the curvature of the part of the cornea eliminated and of addressing its measurements to said computer (34).

15. Apparatus according to either one of claims 13 or 14, characterized in that it comprises an auxiliary laser generator (38) adapted to align said beam (4) with respect to said cornea (1).

Fig.1

Fig.2

Fig.3

*Fig. 4*

*Fig. 5*

*Fig. 6*

*Fig:7*

*Fig:8*

*Fig:9*

*Fig. 10*

Fig.11